# FASCICULE DE BREVET EUROPEEN

(11) **EP 1 921 995 B1**
(45) Date de publication et mention de la délivrance du brevet: **21.07.2010**
(21) Numéro de dépôt: 06763745.4
(22) Date de dépôt: 15.06.2006
(51) Int. Cl.: A61B 6/14

(54) **CAPTEUR D'IMAGE A COINS COUPES**
BILDSENSOR MIT ECKSCHNITTEN
IMAGE SENSOR WITH CORNER CUTS

(30) Priorité: 01.07.2005 FR 0507035
(43) Date de publication de la demande: 21.05.2008
(73) Titulaire: E2V Semiconductors, 38521 Saint Egreve (FR)
(72) Inventeur: LIGOZAT, Thierry, F-38000 Grenoble (FR); CHENEBAUX, Grégoire, F-38000 Grenoble (FR)
(74) Mandataire: Guérin, Michel
(86) Numéro de dépôt international: PCT/EP2006/063251
(87) Numéro de publication internationale: WO 2007/003495

(56) Documents cités:
- EP-A- 1 255 401
- FR-A- 2 857 160
- US-A- 5 291 010
- US-A- 5 510 623
- US-B1- 6 794 654

## Description

L'invention concerne les capteurs d'image destinés à être logés dans des endroits où la place disponible est réduite, et pour lesquels on veut cependant une zone de capture d'image la plus grande possible. C'est le cas par exemple des capteurs d'image radiologique dentaire : ils doivent être logés dans la bouche d'un patient et la dimension de l'image prise doit correspondre au moins à la hauteur d'une dent et à la largeur de plusieurs dents. Les contraintes d'encombrement sont donc fortes et il faut essayer de gagner le plus possible en volume du capteur à surface d'image donnée. De plus, le souci du confort du patient entraîne des contraintes supplémentaires d'ergonomie.

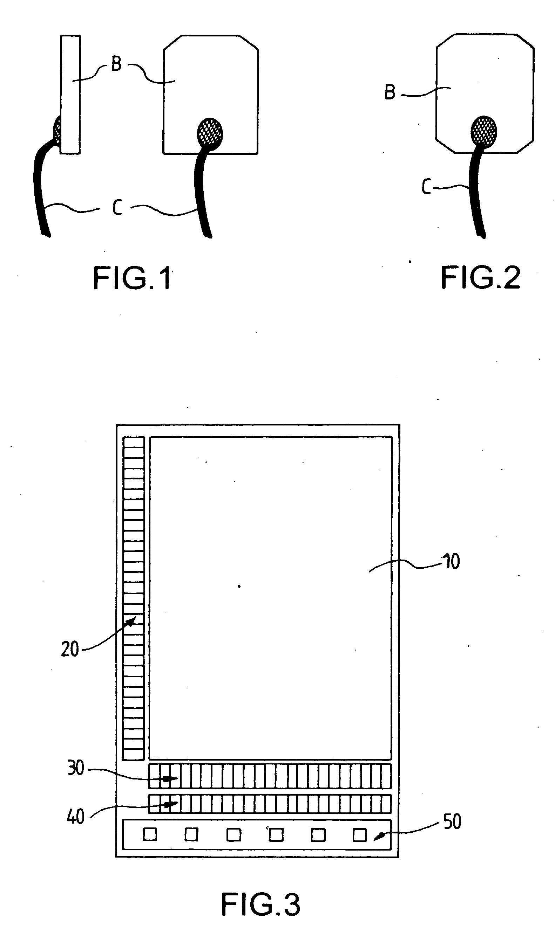

Le capteur d'image radiologique comporte classiquement une puce semiconductrice portant une matrice d'éléments photosensibles et des circuits électroniques associés, une carte de circuit imprimé sur laquelle sont montés la puce et éventuellement quelques autres composants, un scintillateur recouvrant la puce, et parfois une galette de fibres optiques interposée entre le scintillateur et la puce. L'ensemble est enfermé dans un boîtier de résine B (figure 1) d'où peut partir un câble de liaison C vers un système d'exploitation des images recueillies (sauf dans le cas de transmission sans fil, auquel cas une pile est en principe prévue dans le boîtier). Le boîtier épouse le plus étroitement possible la forme de la puce afin de ne pas engendrer un encombrement inutile.

La forme a priori rectangulaire de la puce impose une forme rectangulaire du boîtier, qui n'est pas ergonomique ni confortable pour le patient.

Pour améliorer l'ergonomie et le confort dans les capteurs d'image radiologique réalisés à partir de technologies CCD ("Charge Coupled Devices" ou dispositifs à transfert de charges), on a déjà proposé de couper ou arrondir les coins du boîtier. Et pour ne pas perdre de surface de capture d'image, on a proposé alors d'utiliser une puce ayant elle-même des coins coupés. Des capteurs à puces à deux coins coupés (les deux coins situés vers l'avant dans le sens d'introduction du capteur dans la bouche) ont été proposés (figure 1) ; des capteurs à quatre coins coupés également (figure 2). Cela entraîne des adaptations de structure telles que, par exemple, la mise en place d'un registre de lecture de charges au milieu de la puce plutôt que sur les bords. Ces adaptations sont possibles dans les technologies CCD. Elles ne le sont pas dans les technologies CMOS, c'est-à-dire des technologies dans lesquelles les éléments photosensibles comportent des éléments actifs à base de transistors MOS au niveau de chaque pixel pour convertir les charges photogénérées en tension ou courant, et dans lesquelles les signaux de tension ou courant correspondant à chaque pixel sont transmis sur un conducteur de colonne associé à chaque colonne de pixels.

En effet, ces technologies n'utilisent pas de registre de transfert de charges et on ne pourrait pas placer des systèmes de lecture en plein milieu de la puce sans neutraliser une surface d'image très importante, ce qui n'est pas acceptable.

Cependant, les technologies CMOS sont très intéressantes car elles permettent de réaliser facilement sur une même puce de circuit intégré à la fois une matrice de prise d'image et des circuits électroniques associés (circuits de commande, circuits de recueil de signaux d'image, circuits de traitement d'image, etc.). D'autre part ce sont des technologies qui consomment moins d'énergie, ce qui est avantageux.

C'est pourquoi il existe un besoin pour combiner les avantages de la technologie CMOS et la forme ergonomique de puces à coins coupés.

Une difficulté importante existe cependant pour réaliser cette combinaison. Cette difficulté est expliquée en se référant d'abord à la figure 3 qui représente schématiquement une puce de capteur d'image rectangulaire. L'essentiel de la surface est occupé par une matrice 10 de lignes et colonnes d'éléments actifs photosensibles.

La matrice comprend en pratique, pour chaque ligne de pixels, un ou plusieurs conducteurs de ligne reliant tous les pixels d'une même ligne, et pour chaque colonne de pixels un conducteur de colonne reliant tous les pixels d'une même colonne.

Le fonctionnement d'une telle matrice en technologie CMOS impose la présence
- d'un circuit de sélection de lignes, 20, pour désigner successivement chaque ligne lors d'une opération de lecture d'image ; c'est un circuit purement numérique ; il comprend un bloc de sélection de ligne en regard de chaque ligne ;
- d'un circuit 30 de lecture de courants ou tensions présents sur les conducteurs de colonne pendant l'adressage d'une ligne déterminée, incluant en principe un circuit de mémorisation de ces tensions ou courants pour mémoriser les signaux d'image correspondant à la ligne adressée pendant tout le temps que dure la lecture individuelle de tous les signaux mémorisés correspondant à cette ligne ; les signaux issus des pixels de cette ligne et mémorisés sont eh effet lus séquentiellement sur un conducteur de sortie de la matrice (non représenté) auquel sont reliés les différents blocs de lecture de courant ou tension du circuit 30 (un bloc par colonne) ; le circuit de lecture 30 est un circuit mixte analogique et numérique ;
- d'un circuit de sélection de colonnes 40 pour désigner successivement chaque colonne pendant une opération de lecture d'une ligne qui vient d'être mémorisée ; la désignation d'une colonne permet de transmettre le signal mémorisé correspondant à cette colonne vers la sortie de la matrice ; le circuit de sélection de colonne est un circuit numérique ; il comprend un bloc de sélection associé à chaque bloc de lecture, donc associé à chaque colonne ; ce circuit 40 n'est pas obligatoirement présent, en particulier lorsque le capteur d'image comporte un convertisseur analogique-numérique très rapide en sortie du circuit de lecture 30 ;
- de différents circuits électroniques et de plots d'entrée-sortie de la puce, l'ensemble logé dans un espace désigné sous la référence générale 50 ; ces circuits peuvent inclure notamment un séquenceur pour l'adressage successif des lignes puis des colonnes pour une ligne donnée.

Les circuits de lecture et de sélection sont disposés à la périphérie de la matrice, en regard des lignes d'une part, des colonnes d'autre part. Le circuit de sélection de lignes 20 s'étend le long d'un bord vertical de la matrice, parallèlement aux colonnes, avec un bloc en regard de chaque ligne ; il peut être dédoublé et s'étendre le long des deux bords verticaux. Le circuit de lecture (et mémorisation) 30 s'étend par exemple le long du bord inférieur horizontal de la matrice. Le circuit de sélection de colonnes 40 s'étend aussi le long du bord inférieur horizontal, au-dessous du circuit de lecture 30. L'espace 50 s'étend au-dessous du circuit 40.

Plus précisément, le circuit de sélection de ligne 20 comprend un bus d'adressage de plusieurs conducteurs (non représenté) qui s'étend tout le long d'un bord vertical de la matrice, parallèlement aux colonnes, et un bloc de sélection en regard de chaque ligne. Le bloc de sélection a pour entrées les conducteurs du bus et pour sorties un ou plusieurs conducteurs de ligne qui relient horizontalement tous les pixels de la ligne correspondant au bloc élémentaire.

Le circuit 30 de lecture de courant ou tension et de mémorisation comprend, pour chaque colonne, un bloc de lecture élémentaire qui exécute la fonction de lecture et mémorisation ; ce bloc est placé en regard de cette colonne, et il reçoit comme entrée le conducteur de colonne correspondant à cette colonne. Ce bloc peut comporter un amplificateur tampon, une ou plusieurs capacités associées, et des interrupteurs ; il se comporte comme un échantillonneur bloqueur, c'est-à-dire que dans une première phase il échantillonne une valeur de courant ou tension présente sur le conducteur de colonne, et dans une deuxième phase, il conserve en mémoire la tension échantillonnée jusqu'à la lecture de son contenu (lecture séquentielle, bloc par bloc).

Le circuit de sélection de colonne 40 comprend un bus d'adressage (non représenté) formé de plusieurs conducteurs, qui s'étend le long d'un bord horizontal de la matrice, parallèlement aux lignes, et un bloc de sélection de colonne en regard de chaque colonne ; ce bloc de sélection est un circuit de décodage (mais pourrait être un simple registre à décalage) qui a pour entrées les conducteurs de ce bus d'adressage et qui a pour sortie un signal de commande d'un interrupteur interposé entre le bloc de lecture en courant ou tension associé à la colonne correspondante et le conducteur de sortie de la matrice. Le bus d'adressage de colonne sélectionne un bloc élémentaire et relie au conducteur de sortie la sortie de l'échantillonneur-bloqueur associé à la colonne désignée. Le conducteur de sortie fournit successivement les signaux d'image correspondant à chaque pixel de la matrice, ligne par ligne et pixel par pixel dans la ligne.

Ainsi, pour une ligne adressée par le bus d'adressage de ligne, les signaux de tous les pixels de la ligne sont mémorisés dans les blocs de lecture 30 situés au bas de la matrice, puis ils sont successivement transmis à la sortie sous la commande du bus d'adressage de colonne, avant qu'une nouvelle ligne ne soit adressée.

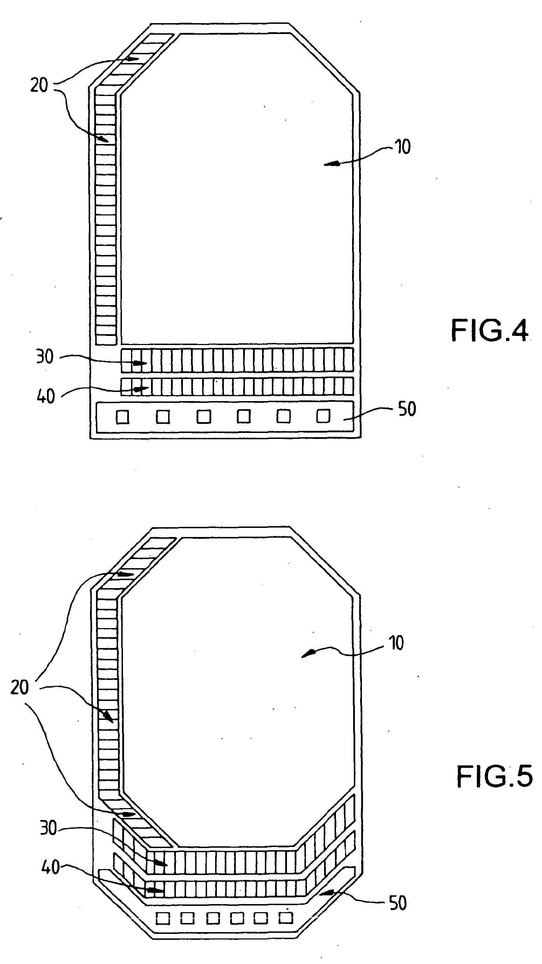

Si maintenant on utilise une matrice à deux ou quatre coins coupés à la place de la matrice rectangulaire de la figure 3, le circuit de sélection de ligne s'étend en partie le long des bords obliques constituant les pans coupés de la matrice pour rester en regard de chaque ligne de la matrice tout en étant logé dans l'espace résiduel étroit entre le bord de la matrice et le bord de la puce. En soi, cela ne pose pas de problème particulier. La figure 4 représente l'implantation générale pour une matrice à deux coins coupés.

Mais dans le cas d'une matrice à quatre coins coupés, le circuit de sélection de colonne et le circuit de lecture de tension ou courant doivent aussi s'étendre en partie le long des coins coupés pour rester en regard des colonnes qui aboutissent sur ces coins. On a constaté que cette disposition pouvait engendrer un motif de bruit fixe ("Fixed Pattern Noise" en anglais ou FPN). En effet, la non-uniformité de position et de réalisation des circuits analogiques engendre des petites différences de gain qui se retrouvent dans l'image sous forme d'un motif de bruit fixe : les circuits de lecture individuels devraient être tous rigoureusement identiques, mais ils ne le sont pas dans la réalité. Il y a un facteur de dépendance entre le gain d'un amplificateur par exemple et la position de l'amplificateur dans la puce. Dans le cas où des amplificateurs sont situés sur une seule ligne, on peut corriger assez facilement l'influence de cette dépendance selon un axe géométrique. Dans le cas où ils sont situés à la fois sur une ligne horizontale (dépendance selon un axe) et sur une ligne oblique (dépendance selon deux axes), cette correction est beaucoup plus difficile et un bruit fixe lié à la structure (et pas seulement aux imperfections technologiques) risque de subsister malgré les corrections.

De plus, sur les coins obliques de la matrice, on ne peut pas avoir comme pour une matrice rectangulaire les blocs de sélection de ligne le long d'un bord horizontal et les blocs de lecture le long d'un bord vertical. Les deux séries sont disposées le long d'un même bord oblique. Il faut alors choisir de placer les blocs de sélection de ligne à proximité immédiate des lignes ou les blocs de lecture à proximité immédiate des colonnes mais on ne peut pas avoir les deux à la fois. Or dans les deux cas cela oblige à faire passer des signaux d'adressage de ligne au-dessus des blocs de lecture. Ces signaux sont des signaux numériques d'amplitude importante qui exercent une influence capacitive difficilement acceptable sur les blocs de lecture qui sont très sensibles aux influences capacitives (ce sont des circuits analogiques de mesure de très faibles courants et tensions). Il faudrait donc interposer des couches de blindage entre les lignes transportant des signaux numériques et les blocs de lecture ; c'est difficilement faisable compte-tenu du nombre limité de niveaux conducteurs disponibles à l'endroit des blocs de lecture (ces blocs utilisent en principe tous les niveaux conducteurs que la technologie employée met à leur disposition).

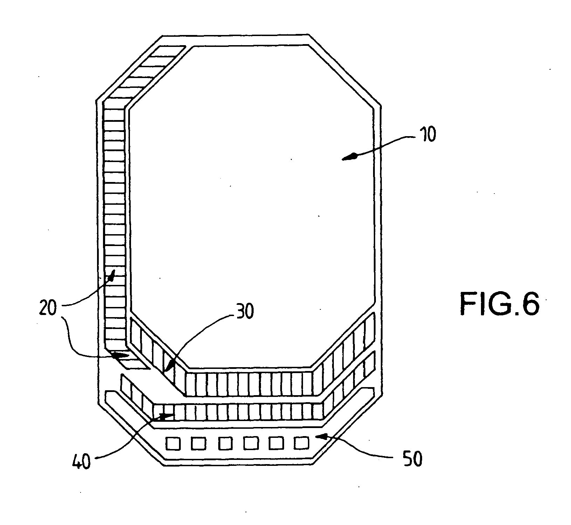

Les figures 5 et 6 représentent deux exemples de structures illustrant les positions possibles des circuits de sélection et de lecture dans une matrice à quatre coins coupés : blocs de sélection de ligne 20 à proximité immédiate du pan coupé de la matrice à la figure 5, blocs de lecture en courant ou tension 30 à proximité immédiate du pan coupé à la figure 6. La demande européenne EP 1 255 401 A1 décrit le préambule de la revendication 1.

Pour résoudre les difficultés liées à ces structures, l'invention propose un capteur d'image à puce à coins coupés, comportant une matrice de lignes horizontales et colonnes verticales d'éléments photosensibles, la matrice ayant une forme généralement rectangulaire de largeur horizontale L et ayant quatre pans coupés, le capteur comportant autant de blocs de lecture de courant ou tension qu'il y a de colonnes de la matrice, pour lire les signaux d'image détectés par les éléments photosensibles d'une colonne et transmis par un conducteur de colonne associé à cette colonne, caractérisé en ce que les blocs de lecture de courant ou tension sont disposés le long d'un bord horizontal de la matrice et tous logés à l'intérieur d'une bande verticale dont la largeur L1 est sensiblement inférieure à la largeur maximale L de la matrice.

En pratique, si L' est la largeur du bas de la matrice, c'est-à-dire la largeur horizontale restreinte qui subsiste entre les pans coupés en bas de la matrice (du côté où sont situés les blocs de lecture de courant ou tension), on fera tenir la totalité des blocs dans la largeur L' ou dans une largeur à peu près égale à la largeur L'.

Si le capteur comporte des blocs de sélection de colonne associés aux blocs de lecture, ces blocs de sélection de colonne seront logés tous dans la même largeur L1.

Dans une première réalisation, les blocs de lecture sont disposés tous avec le même pas (de préférence le pas des colonnes auxquels ils sont associés), et les blocs qui sont associés aux colonnes aboutissant le long des pans coupés sont situés au-dessous de blocs associés à des colonnes aboutissant le long du bord horizontal de la matrice. Les blocs de lecture sont disposés en deux rangées situées l'une au-dessous de l'autre.

Dans une deuxième réalisation, les blocs de lecture sont disposés le long d'un bord horizontal de la matrice avec un pas entre blocs plus petit que le pas des colonnes qui leur sont associées. Tous les blocs de lecture sont logés sur une seule rangée, dans une largeur L1 qui est sensiblement inférieure à la largeur maximale de la matrice. Le rapport entre le pas des blocs de lecture et le pas des pixels est de préférence à peu près égal à L'/L, de sorte que les blocs tiennent pour l'essentiel dans la largeur L' du bas de la matrice.

D'autres caractéristiques et avantages de l'invention apparaîtront à la lecture de la description détaillée qui suit et qui est faite en référence aux dessins annexés dans lesquels :
- les figures 1 à 3 déjà décrites représentent des structures connues de capteurs d'image ;
- la figure 4 représente une implantation générale de circuits possible pour un capteur à deux coins coupés ;
- les figures 5 et 6 représentent des implantations générales possibles pour des capteurs à quatre coins coupés ;
- la figure 7 représente un capteur selon l'invention dans un premier mode de réalisation ;
- la figure 8 représente un capteur selon l'invention dans un deuxième mode de réalisation ;
- la figure 9 représente un détail de structure de la réalisation de la figure 8.

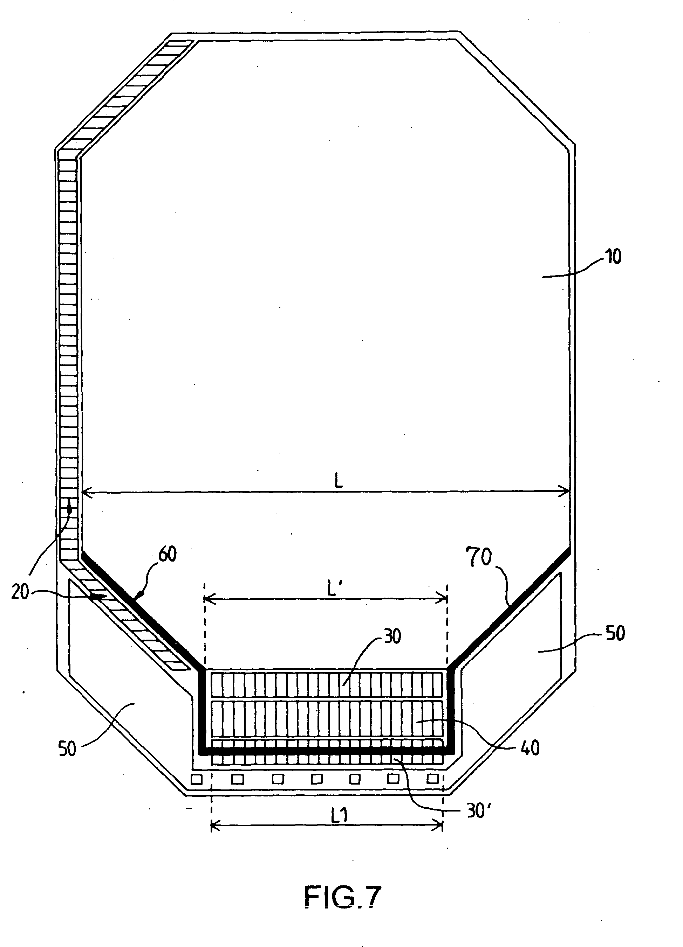

L'implantation générale des circuits du capteur selon l'invention est représentée à la figure 7. Les références identiques à celles des figures précédentes correspondent à des éléments ayant les mêmes fonctionnalités.

La matrice 10 possède quatre coins coupés ; les coins sont coupés en principe à 45° et ils sont coupés sur une part substantielle de la matrice. Par exemple, si la matrice comporte N colonnes au total dans sa plus grande largeur et N' colonnes à sa base horizontale, on peut envisager que N' soit pratiquement la moitié de N, ce qui veut dire que les coins coupés s'étendent chacun sur à peu près un quart des colonnes de la matrice.

On appelle L la largeur totale de la matrice et L' la largeur de la base horizontale (le terme horizontal correspond à la direction parallèle aux lignes d'adressage, le terme vertical correspond à la direction parallèle aux colonnes qui fournissent les signaux recueillis). L' peut alors être égal à L/2 environ.

Les circuits de sélection de ligne 20 s'étendent à la fois le long d'un bord vertical de la matrice et le long des bords obliques adjacents à ce bord vertical. Ils sont représentés sous forme d'une série de rectangles et parallélogrammes juxtaposés les uns au-dessus des autres, chaque rectangle (le long du bord vertical) ou parallélogramme (le long des bords obliques) correspondant à un bloc de sélection d'une ligne respective de la matrice et étant en regard de cette ligne. Pour des raisons de symétrie on peut prévoir que les circuits de sélection de ligne s'étendent aussi sur l'autre bord vertical et les deux autres bords obliques. La symétrie est par rapport à un axe vertical médian coupant la matrice en deux. Cette disposition est facultative. Elle est utile pour des raisons de centrage, de redondance et de réduction du temps d'accès aux pixels centraux de la matrice. Elle n'est pas représentée.

Selon l'invention, le circuit de lecture de tension ou courant est constitué d'une série de blocs individuels (un bloc par colonne de la matrice) qui sont disposés côte à côte sur une ou deux rangées horizontales, à l'intérieur d'une largeur L1, mesurée dans le sens horizontal, qui est plus nettement plus courte que la largeur L de la matrice, et qui est de préférence proche de la largeur L' de la base horizontale de la matrice. Les blocs individuels sont représentés par des rectangles juxtaposés correspondant chacun à une colonne respective.

Sur la figure 7, les blocs de lecture sont disposés sur deux rangées horizontales 30 et 30 et à l'intérieur d'une bande verticale de largeur L1.

Tous les blocs de lecture individuels associés aux colonnes qui débouchent sur la base horizontale de la matrice sont disposés en une première rangée 30 immédiatement sous cette base, un bloc étant situé au-dessous de chaque colonne, et le pas de répartition des blocs est le même que le pas de répartition des colonnes, c'est-à-dire le même que le pas des pixels de la matrice dans la direction horizontale. Tous les autres blocs de lecture, qui sont donc associés aux colonnes qui débouchent sur les pans coupés de la matrice, sont disposés en une deuxième rangée horizontale 30' au-dessous des blocs de la première rangée et avec le même pas. Les blocs des deux rangées sont de préférence identiques les uns aux autres ; ils peuvent cependant être disposés symétriquement par rapport à ceux de la première rangée (symétrie par rapport à une ligne horizontale).

S'il y a un circuit de sélection de colonnes 40 (dans le cas général), il est situé entre les deux rangées de blocs de lecture 30 et 30'.

Les conducteurs de colonne issus des colonnes aboutissant sur l'un des pans coupés (celui de gauche sur la figure) sont regroupés en un bus 60 de multiples conducteurs (autant de conducteurs qu'il y a de colonnes aboutissant sur le pan coupé) qui s'étend le long de ce pan coupé puis qui longe verticalement la rangée 30 et le circuit de sélection de colonne 40, et qui va jusqu'aux blocs de lecture de la moitié de gauche de la deuxième rangée 30'. Ce bus est de préférence intercalé entre les blocs de décodage de ligne 20 situés le long des pans coupés et le bord oblique de la matrice. Les conducteurs de sélection de ligne croisent alors le bus de conducteurs de colonnes et on prévoit de préférence qu'une couche conductrice de blindage, portée à un potentiel fixe, est intercalée entre les conducteurs de ligne et les conducteurs de colonne là où ils se croisent afin d'éviter que les signaux numériques appliqués aux lignes n'influencent capacitivement les potentiels analogiques des conducteurs de colonne, ce qui perturberait la lecture. Ces croisements se font là où il n'y a pas de blocs de lecture ; par conséquent il est facile de les réaliser technologiquement avec seulement deux niveaux de conducteur et un niveau isolant entre eux.

De l'autre côté de la matrice, sur le pan coupé de droite, les conducteurs de colonne sont regroupés en un autre bus 70 de multiples conducteurs. Ce bus 70 longe le pan coupé puis descend le long des circuits 30 et 40 pour rejoindre la moitié droite de la rangée 30' de blocs de lecture.

La zone 50 servant à loger les plots d'entrée-sortie et d'autres circuits fonctionnels s'étend au bas de la puce, aussi bien sous le bord inférieur horizontal de la matrice que sous les bords obliques, de manière à optimiser le taux de remplissage.

Les blocs de lecture de courant ou tension (30, 30') sont disposés parallèlement les uns aux autres et n'engendrent pas de motif de bruit fixe (FPN) significatif et non maîtrisable qui serait lié à la structure à deux dimensions. Quant aux circuits de sélection de ligne (20), ils ne manipulent que des données binaires et ils ne sont donc pas sensibles au bruit fixe lié à la structure à deux dimensions qui résulterait de leurs positions en colonne verticale et le long de pans coupés.

Le bloc 30' n'occupe pas forcément toute la largeur L1 de la base horizontale de la matrice.

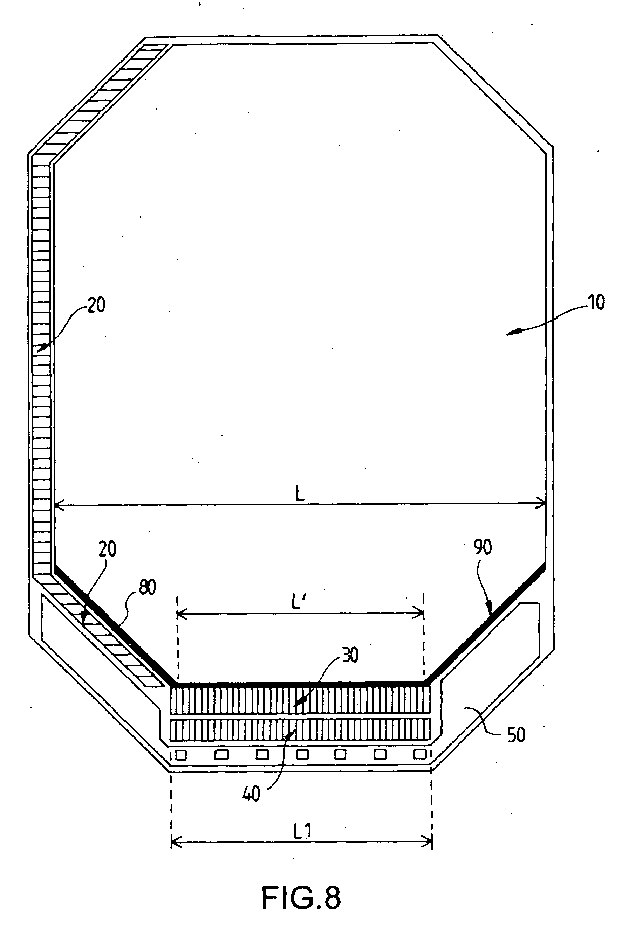

La figure 8 représente une autre réalisation dans laquelle tous les blocs de lecture sont disposés côte à côte sur une seule rangée horizontale 30, à l'intérieur d'une largeur L1 qui est inférieure à L et qui est de préférence à peu près la largeur L' de la base horizontale de la matrice. Les blocs sont juxtaposés avec un pas qui est plus petit que le pas des colonnes de la matrice c'est-à-dire plus petit que le pas des pixels d'une ligne. Le pas est dans un rapport L1/L (en pratique environ L'/L, et par exemple 1/2 si L'=L/2) avec le pas des colonnes de pixels pour que tous les blocs de lecture tiennent dans la largeur L1. Si le pas des colonnes est d'environ 10 micromètres, il est facile de faire tenir un bloc de lecture de courant ou tension comprenant quelques transistors dans un pas de 5 micromètres.

S'il y a un circuit de sélection de colonne 40, il est situé sous la rangée 30 de blocs de lecture et les blocs de sélection de colonne sont au même pas que les blocs de lecture.

Un bus de conducteurs de colonnes 80 (à gauche) et un bus de conducteurs de colonnes 90 (à droite) s'étendent le long d'un bord oblique respectif (entre la matrice et les décodeurs de ligne pour le bus 80 de gauche) et le long du bord inférieur horizontal de la matrice (entre la matrice et les blocs de lecture). Ils ramènent chacun des conducteurs de colonne, qu'il vienne d'un pan coupé à gauche ou à droite ou qu'il vienne du bord inférieur de la matrice, vers un bloc de lecture correspondant de la rangée unique 30. Ces bus sont nécessaires car les blocs de lecture ne sont pas situés au-dessous des colonnes de pixels de la matrice, même pour les colonnes qui arrivent sur le bord horizontal, du fait que le pas des blocs de lecture n'est pas le même que le pas des colonnes de pixels de la matrice ; seules une ou deux colonnes centrales de la matrice débouchent au-dessus des blocs correspondants.

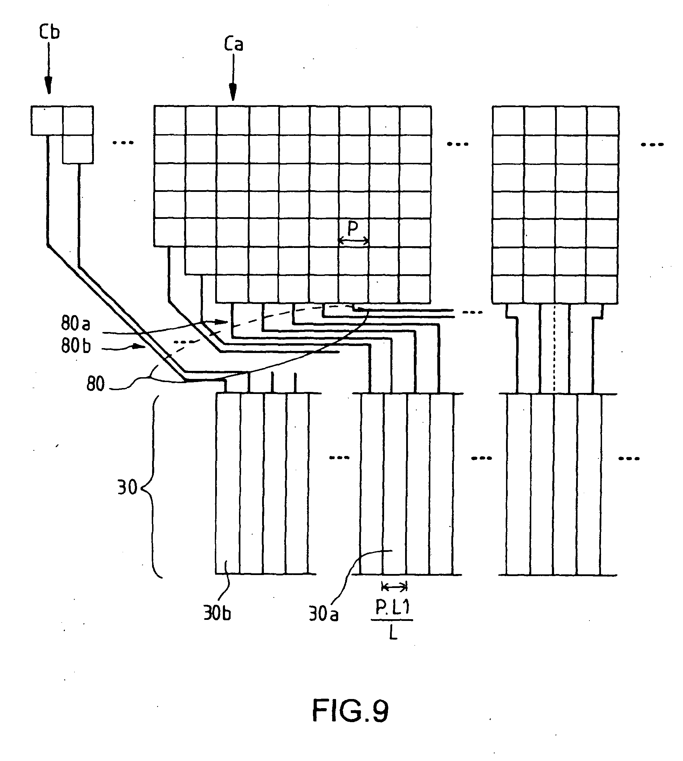

La figure 9 représente une vue agrandie de la disposition de la figure 8, pour mieux faire comprendre la constitution du bus de conducteurs 80. Il comprend des conducteurs tels que 80a, provenant d'une colonne Ca aboutissant sur le bord inférieur horizontal de la matrice, ce conducteur 80a allant vers un bloc de lecture 30a qui est situé sous le bord inférieur horizontal de la matrice mais qui n'est pas situé en regard de la colonne Ca. Et il comprend des conducteurs tels que 80b, provenant d'une colonne Cb aboutissant sur un bord oblique, ces conducteurs 80b allant vers un bloc de lecture 30b qui est situé sous le bord inférieur horizontal de la matrice. Les conducteurs 80 peuvent être réalisés avec un ou plusieurs niveaux de métallisation, l'utilisation de plusieurs niveaux superposés permettant de réduire la largeur du bus.

Le pas des colonnes de pixels est P. Le pas des blocs de lecture est P.(L1/L), plus petit que P.

L'invention est applicable tout particulièrement aux capteurs d'image radiologique dentaire intraoraux pour lesquels l'encombrement et le confort pour le patient sont des paramètres importants.

La puce peut avoir dans ce cas des dimensions de l'ordre de 20 mm de côté, par exemple une largeur L de 20mm et une largeur de base L' de 10 millimètres, soit environ la moitié.

## Revendications

1. Capteur d'image à puce de circuit intégré rectangulaire à coins coupés, comportant une matrice (10) de lignes horizontales et de colonnes verticales d'éléments photosensibles, la matrice ayant une forme généralement rectangulaire de largeur horizontale L et ayant quatre pans coupés, le capteur comportant autant de blocs de lecture de courant ou tension qu'il y a de colonnes de la matrice, pour lire les signaux d'image détectés par les éléments photosensibles d'une colonne et transmis par un conducteur de colonne associé à cette colonne, les blocs de lecture de courant ou tension étant disposés en rangée (30, 30') le long d'un bord horizontal, de largeur L', de la matrice (10), **caractérisé en ce que** lesdits blocs de lecture de courant ou tension sont tous logés à l'intérieur d'une bande verticale dont la largeur L1 dans le sens horizontal est sensiblement inférieure à la largeur maximale L de la matrice.

2. Capteur d'image selon la revendication 1, **caractérisé en ce que** ladite largeur L1 de ladite bande verticale est sensiblement égale à la largeur L' du bord horizontal de la matrice entre les pans coupés de la matrice.

3. Capteur selon l'une des revendications 1 et 2, **caractérisé en ce que** les blocs de lecture sont disposés en deux rangées horizontales (30, 30' ; fig. 7) situées l'une au-dessous de l'autre, les blocs qui sont associés aux colonnes aboutissant le long des pans coupés étant situés au-dessous de blocs associés à des colonnes aboutissant le long du bord horizontal de la matrice et le pas des blocs étant le même dans les deux rangées.

4. Capteur selon la revendication 3, **caractérisé en ce que** le pas des blocs de lecture est le même que celui des colonnes auxquels ils sont associés.

5. Capteur selon la revendication 3, **caractérisé en ce qu'**un bus conducteur (60) s'étend le long d'un pan coupé pour relier les conducteurs de colonne aboutissant sur ce pan coupé à la deuxième rangée (30') de blocs de lecture.

6. Capteur selon la revendication 5, **caractérisé en ce que** le bus conducteur (60) s'étend le long du pan coupé entre la matrice et des blocs de sélection de ligne (20) servant à la sélection des lignes d'image.

7. Capteur selon l'une des revendications 3 à 6, **caractérisé en ce qu'**il comporte une rangée de blocs de sélection de colonne (40) placée entre les deux rangées de blocs de lecture (30, 30'), chaque bloc de sélection de colonne servant à la sélection d'un bloc de lecture associé à une colonne respective.

8. Capteur selon l'une des revendications 1 et 2, **caractérisé en ce** les blocs de lecture de tension ou courant sont disposés le long d'un bord horizontal de la matrice avec un pas entre blocs plus petit que le pas des colonnes qui leur sont associées, tous les blocs de lecture étant logés sur une seule rangée (30, fig. 8), dans une largeur L1 qui est sensiblement inférieure à la largeur maximale de la matrice.

9. Capteur selon la revendication 8, **caractérisé en ce qu'**un bus conducteur (60) s'étend le long d'un pan coupé et du bord horizontal de la matrice pour relier les conducteurs de colonne aboutissant sur ce pan coupé d'une part, et sur le bord horizontal d'autre part, aux blocs de lecture.

10. Capteur selon la revendication 9, **caractérisé en ce que** le bus conducteur (60) s'étend le long du pan coupé entre la matrice et des blocs de sélection de ligne (20) servant à la sélection des lignes d'image.

11. Capteur selon l'une des revendications 8 à 10, **caractérisé en ce qu'**il comporte une rangée de blocs de sélection de colonne (40) servant à la sélection des blocs de lecture en tension ou en courant, cette rangée étant placée au-dessous de la rangée de blocs de lecture (30) et les blocs de sélection de colonne étant au même pas que les blocs de lecture en tension ou en courant.

12. Capteur d'image selon l'une des revendications 1 à 11, **caractérisé en ce qu'**il constitue un capteur d'image radiologique dentaire intraoral.

## Claims

1. An image sensor having a rectangular integrated circuit chip with cut corners, comprising a matrix (10) of horizontal lines and vertical columns of photosensitive members, the matrix having a generally rectangular shape of horizontal width L and having four bevels, the sensor comprising as many current or voltage read blocks as there are matrix columns, in order to read the image signals detected by the photosensitive members of a column and transmitted by a column conductor linked with this column, the current or voltage read blocks being placed in a row (30, 30') along a horizontal edge, of width L', of the matrix (10), **characterized in that** all said current or voltage read blocks are housed within a vertical strip, the width L1 of which, in the horizontal direction, is substantially less than the maximum width L of the matrix.

2. The image sensor as claimed in claim 1, **characterized in that** said width L1 of said vertical strip is substantially equal to the width L' of the horizontal edge of the matrix between the bevels of the matrix.

3. The sensor as claimed in either of claims 1 and 2, **characterized in that** the read blocks are placed in two horizontal rows (30, 30'; Fig. 7), one located below the other, the blocks which are linked with the columns terminating along the bevels being located below blocks linked with columns terminating along the horizontal edge of the matrix and the pitch of the blocks being the same in both rows.

4. The sensor as claimed in claim 3, **characterized in that** the pitch of the read blocks is the same as that of the columns with which they are linked.

5. The sensor as claimed in claim 3, **characterized in that** a conductor bus (60) extends along a bevel in order to connect the column conductors terminating on this bevel to the second read block row (30').

6. The sensor as claimed in claim 5, **characterized in that** the conductor bus (60) extends along the bevel between the matrix and line select blocks (20) used to select the image lines.

7. The sensor as claimed in one of claims 3 to 6, **characterized in that** it comprises a column select block row (40) which is positioned between the two read block rows (30, 30'), each column select block being used to select a read block linked with a respective column.

8. The sensor as claimed in either of claims 1 and 2, **characterized in that** the current or voltage read blocks are placed along a horizontal edge of the matrix with a pitch between blocks which is smaller than the pitch of the columns with which they are linked, all of the read blocks being housed in a single row (30, Fig. 8), in a width L1 which is substantially less than the maximum width of the matrix.

9. The sensor as claimed in claim 8, **characterized in that** a conductor bus (60) extends along a bevel and the horizontal edge of the matrix in order to connect the column conductors terminating on this bevel on the one hand, and on the horizontal edge on the other, to the read blocks.

10. The sensor as claimed in claim 9, **characterized in that** the conductor bus (60) extends along the bevel between the matrix and line select blocks (20) used to select the image lines.

11. The sensor as claimed in one of claims 8 to 10, **characterized in that** it comprises a column select block row (40) used to select the current or voltage read blocks, this row being positioned below the read block row (30) and the column select blocks being at the same pitch as the current or voltage read blocks.

12. The image sensor as claimed in one of claims 1 to 11, **characterized in that** it makes up an intraoral dental radiological image sensor.

## Patentansprüche

1. Rechteckiger IC-Chip-Bildsensor mit Eckschnitten, der eine Matrix (10) von horizontalen Linien und vertikalen Spalten von photosensiblen Elementen umfasst, wobei die Matrix eine allgemein rechteckige Form mit der horizontalen Breite L und vier geschnittenen Feldern hat, wobei der Sensor so viele Strom- oder Spannungsleseblöcke hat, wie es Matrixspalten gibt, um die von den photosensiblen Elementen einer Spalte erfassten und von einem mit dieser Spalte assoziierten Spaltenleiter übertragenen Bildsignale zu lesen, wobei die Strom- oder Spannungsleseblöcke in einer Reihe (30, 30') entlang einem horizontalen Rand der Breite L' der Matrix (10) angeordnet sind, **dadurch gekennzeichnet, dass** sich alle Strom- oder Spannungsleseblöcke innerhalb eines vertikalen Streifens befinden, dessen Breite L1 in der horizontalen Richtung erheblich geringer ist als die maximale Breite L der Matrix.

2. Bildsensor nach Anspruch 1, **dadurch gekennzeichnet, dass** die Breite L1 des vertikalen Streifens im Wesentlichen gleich der Breite L' des horizontalen Randes der Matrix zwischen den geschnittenen Feldern der Matrix ist.

3. Bildsensor nach einem der Ansprüche 1 und 2, **dadurch gekennzeichnet, dass** die Leseblöcke in zwei untereinander liegenden horizontalen Reihen (30, 30'; Fig. 7) angeordnet sind, wobei sich die mit den Spalten assoziierten Blöcke, die an die geschnittenen Felder anstoßen, unter Blöcken befinden, die mit Spalten assoziiert sind, die entlang dem horizontalen Rand der Matrix anstoßen, und wobei der Blockschritt in den beiden Reihen gleich ist.

4. Bildsensor nach Anspruch 3, **dadurch gekennzeichnet, dass** der Schritt der Leseblöcke derselbe ist wie bei den mit diesen assoziierten Spalten.

5. Bildsensor nach Anspruch 3, **dadurch gekennzeichnet, dass** ein Leitungsbus (60) entlang einem geschnittenen Feld verläuft, um die Spaltenleiter, die an dieses geschnittene Feld anstoßen, mit der zweiten Reihe (30') von Leseblöcken zu verbinden.

6. Bildsensor nach Anspruch 5, **dadurch gekennzeichnet, dass** der Leitungsbus (60) entlang dem geschnittenen Feld zwischen der Matrix und Linienauswahlblöcken (20) verläuft, die zum Auswählen der Bildlinien dienen.

7. Bildsensor nach einem der Ansprüche 3 bis 6, **dadurch gekennzeichnet, dass** er eine Reihe von Spaltenauswahlblöcken (40) umfasst, die sich zwischen den beiden Leseblockreihen (30, 30') befinden, wobei jeder Spaltenauswahlblock zum Auswählen eines mit einer jeweiligen Spalte assoziierten Leseblocks dient.

8. Bildsensor nach einem der Ansprüche 1 und 2, **dadurch gekennzeichnet, dass** die Spannungs- oder Stromleseblöcke entlang einem horizontalen Rand der Matrix mit einem Schritt zwischen Blöcken angeordnet sind, der kleiner ist als der Schritt der mit diesen assoziierten Spalten, wobei sich alle Leseblöcke auf einer einzigen Reihe (30, Fig. 8) in einer Breite L1 befinden, die wesentlich geringer ist als die maximale Breite der Matrix.

9. Bildsensor nach Anspruch 8, **dadurch gekennzeichnet, dass** ein Leitungsbus (60) entlang einem geschnittenen Feld und dem horizontalen Rand der Matrix verläuft, um die Spaltenleiter, die an dieses geschnittene Feld einerseits und an den horizontalen Rand andererseits anstoßen, mit den Leseblöcken zu verbinden.

10. Bildsensor nach Anspruch 9, **dadurch gekennzeichnet, dass** der Leitungsbus (60) entlang dem geschnittenen Feld zwischen der Matrix und Linienauswahlblöcken (20) verläuft, die zum Auswählen der Bildlinien dienen.

11. Bildsensor nach einem der Ansprüche 8 bis 10, **dadurch gekennzeichnet, dass** er eine Reihe von Spaltenauswahlblöcken (40) umfasst, die zum Auswählen der Spannungs- oder Stromleseblöcke dienen, wobei sich diese Reihe unter der Leseblockreihe (30) befindet und die Spaltenauswahlblöcke denselben Schritt haben wie die Spannungs- oder Stromleseblöcke.

12. Bildsensor nach einem der Ansprüche 1 bis 11, **dadurch gekennzeichnet, dass** er einen radiologischen intraoralen Gebissbildsensor darstellt.
